# EUROPEAN PATENT SPECIFICATION

(11) **EP 2 067 849 B1**
(45) Date of publication and mention of the grant of the patent: **23.05.2012**
(21) Application number: 08170395.1
(22) Date of filing: 01.12.2008
(51) Int. Cl.: C12M 1/00

(54) **Incubator**
Inkubator
Incubateur

(30) Priority: 03.12.2007 JP 2007312934
(43) Date of publication of application: 10.06.2009
(73) Proprietor: Sanyo Electric Co., Ltd., Osaka 570-8677 (JP)
(72) Inventor: Watanabe, Kiyoshi, Gunma-ken (JP)
(74) Representative: Grey, Ian Michael

(56) References cited:
- EP-A- 0 154 536
- DE-A1-102006 022 652
- US-A- 3 848 569
- US-A- 3 987 133
- US-A1- 2006 194 193
- US-A1- 2008 057 568

## Description

The present invention relates to an incubator such as a CO2 incubator in which a humidifying water dish portion is provided with the water level detecting device using a self-heating type thermistor.

As shown in Japanese Patent No. 3197696, in an incubator such as a CO2 incubator, humidifying water is accommodated in a bottom portion of a storeroom to maintain a predetermined high humidity of the storeroom and the water is heated by a heater.

In addition, as shown in Japanese Patent No. 3670876 , in such an incubator, the humidifying water is irradiated and sterilized by ultraviolet rays by the use of an ultraviolet lamp to suppress the propagation of bacteria.

When the humidifying water is evaporated, the humidity in the storeroom is lowered and thus a culture medium is affected. For this reason, it is considered to provide a water level detecting device for detecting a water level of the humidifying water. When the water level detecting device is a float type device, the cleaning and the like are troublesome.

As the water level detecting device, a water level detecting device using a self-heating type thermistor has been proposed (see Japanese Patent Application Laid-Open No. 2001-159556 and Japanese Patent Application Laid-Open No. 7-260547).

The present invention relates to an incubator for incubating a culture medium, the incubator comprising a water reservoir, detection means for detecting when the amount of water present in the water reservoir has dropped below a predetermined level and means for supplying water to said reservoir in response to a signal from said detection means to indicate that the water is below said predetermined level. Such an incubator is known from EP 0 154 536A.

The invention relates to the improvement of an incubator.

An incubator according to the present invention is characterised in that said means for supplying water is configured to supply water at a reduced rate whilst a culture medium is being incubated.

Preferably, the incubator further comprises a heater configured to control the temperature of water in the water reservoir.

Advantageously, the heater is configured to stop or reduce the heat provided when the means for supplying water supplies water to the water reservoir.

Conveniently, the water reservoir is disposed in the lower portion of the incubator.

In one embodiment, said means for supplying water is configured to supply it intermittently until said predetermined level has been reached, whilst a culture medium is being incubated.

The incubator may include control means operable to control the supply of water to said water reservoir.

The incubator may further comprise a notification means configured to notify a user when the amount of water present in the water reservoir has dropped below a predetermined level.

Advantageously, the notification means is configured to store and notify a history of when the amount of water present in the water reservoir has dropped below a predetermined level.

The incubator may further comprise an ultraviolet ray radiation means to radiate ultraviolet rays for sterilizing the water; and a temperature sensor to measure a temperature of the water, which is protected from the ultraviolet rays by a tube.

Preferably, the temperature sensor comprises a first self-heating type thermistor and a second self-heating type thermistor, the level of self heating of the second thermistor being less than the level of self heating of the first thermistor, and the thermistors being provided at a position corresponding to a predetermined water level of the water reservoir, wherein the incubator further comprises a power source configured to supply power to the thermistors intermittently, and a control means configured to compare the output voltages corresponding to the temperatures measured by the thermistors and to detect that the water level of the water reservoir is below the predetermined water level when the voltage of the first thermistor is less than the voltage of the second thermistor.

According to another aspect of the invention, there is provided a method of incubating a culture medium in an incubator comprising a water reservoir, the method including the steps of detecting when the amount of water present in the water reservoir has dropped below a predetermined level and supplying water to said reservoir in response to a signal from a detection means indicating that the water level has fallen below said predetermined level.

The invention according to another aspect of the invention is characterised by the method including the step of supplying water to the water reservoir at a reduced rate when a culture medium is being incubated in the incubator.

Preferably, the method includes the step of supplying water to the water reservoir intermittently over a period of time, when a culture medium is being incubated in the incubator.

According to oneaspect of the invention, in an incubator incubating a culture medium accommodated in an incubation space defined in a storage comprising a heater (17) to control a temperature of the water stored in a water storing structure (30) which is in the bottom side of the storage, and to keep the temperature of the water a predetermined temperature; and a water supplier (16) to supply the water to the water storing structure when the water has been decreased. In the incubator when the water supplier (16) supplies the water during the culture medium is incubated, the water supplier decreases the amount of water supplied per unit time than the amount of water supplied during the culture medium is not incubated. That is, when the incubation is not performed, it doesn't matter if a temperature and a humidity vary and thus the water is supplied to shorten the amount of time. However, during the incubation, the water is slowly supplied to suppress the variation in temperature and humidity.

According to another aspect of the invention, the water supplier (16) supplies the water intermittently. In this manner, when the water is supplied, it is easier to control a valve to be completely opened and completely closed than to control the fastening amount of the valve in order to slowly supply the water.

According to another aspect of the invention, in an incubator, the heating operation performed by the heater (17) is stopped or weakened when the water supplier is supplying the water. In this manner, no-water heating is prevented.

According to another aspect of the invention, in an incubator including a notification means (12, 13, 14) to notify that the water level fell down than the predetermined water level during incubation.

According to another aspect of the invention, in an incubator, the notification means (display portion 12) further stores and notifies that a history of the notification.

According to another aspect of the invention, in an incubator further comprising an ultraviolet rays radiation means to radiate ultraviolet rays for sterilizing the water and a temperature sensor (2, 3) to measure a temperature of the water, which is protected from the ultraviolet rays by a tube (2C).

According to another aspect of the invention, in an incubator, the temperature sensor comprising a first self-heating type thermistor (2) and a second self-heating type thermistor (3) which amount of self-heating is less than that of the first self-heating type thermistor (2), the thermistors are provided at a position corresponds to a predetermined water level of the water storing structure. The incubator further comprising a power source to supply power to the thermistors intermittently and a judging means comparing the voltages correspond to the temperatures measured by the thermistors, and judging the water level of the water storing structure fell down than the predetermined water level when the voltage of the first self-heating type thermistor (2) is less than the voltage of the second self-heating type thermistor (3) having less amount of self-heating.

### BRIEF DESCRIPTION OF THE DRAWINGS

Fig. 1 is a schematic circuit diagram of a first embodiment of an incubator according to the invention;
Fig. 2 is a diagram for illustrating output voltage values in accordance with temperatures of thermistors of the first embodiment;
Fig. 3 is a diagram of the thermistor which is a temperature sensor of the first embodiment;
Fig. 4 is a partially fractured perspective view for illustrating the case where the thermistors of the first embodiment are attached to the humidifying dish; and
Fig. 5 is a timing chart for illustrating intermittent driving of the thermistor of the first embodiment.

### DETAILED DESCRIPTION OF THE PREFERRED EMBODIMENTS

### [First Embodiment]

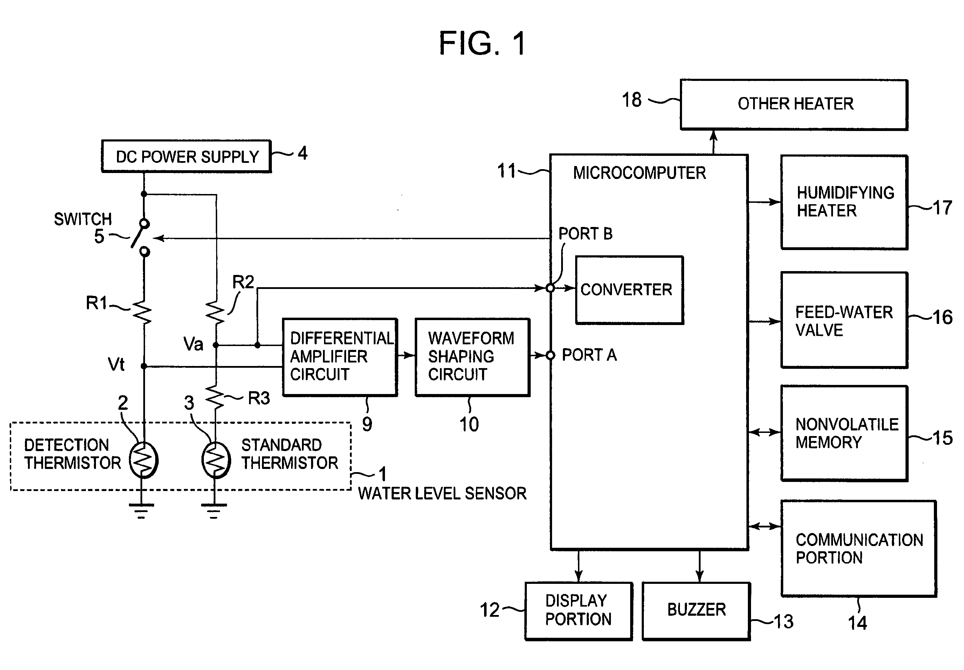

A first embodiment of the invention will be described with reference to Figs. 1 to 5.

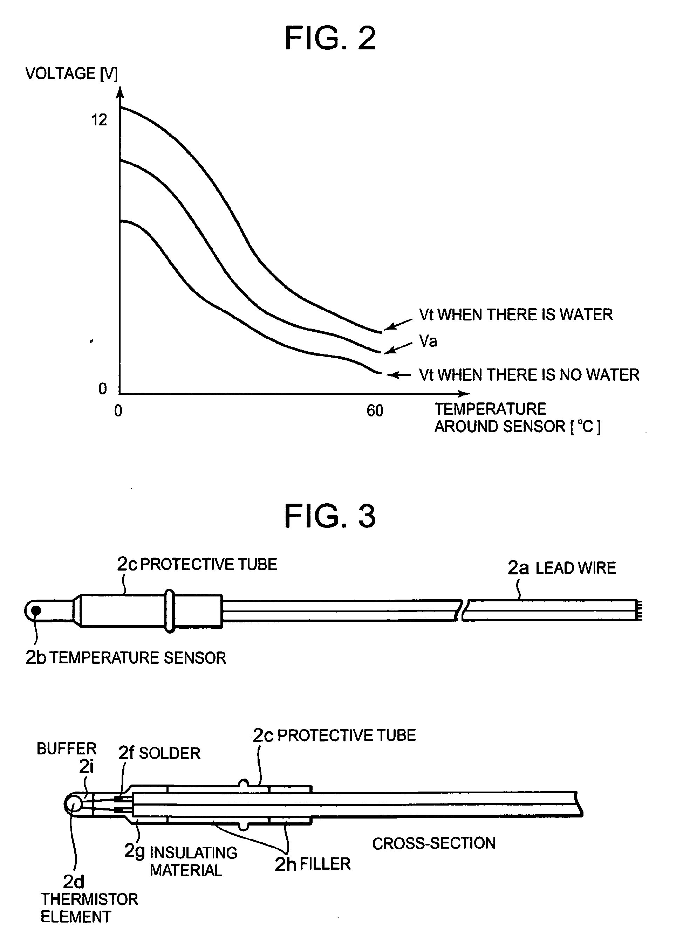

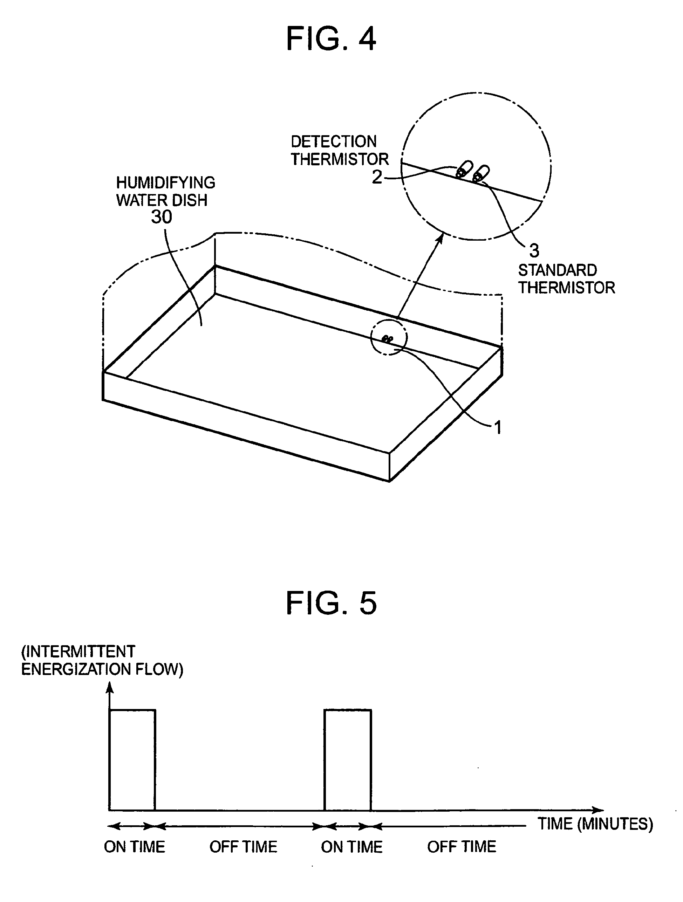

The first embodiment is an incubator employing a water level detecting device according to the invention. Fig. 1 is a schematic circuit diagram of the incubator. Fig. 2 is a diagram for illustrating output voltage values in accordance with temperatures of thermistors. Fig. 3 is a diagram of the thermistor which is a temperature sensor. Fig. 4 is a partially fractured perspective view for illustrating the case where the thermistors are attached to the humidifying dish portion. Fig. 5 is a timing chart for illustrating intermittent driving of the thermistor.

In Fig. 1, reference numeral 1 is a water level sensor. The water level sensor 1 is provided in the humidifying dish portion 30 (water storing structure) on an inner bottom surface of an incubator for incubating a culture medium accommodated in an incubation space defined in a storage to detect water in the humidifying dish portion 30. Reference numeral 2 is a detection thermistor (first self-heating type thermistor) and reference numeral 3 is a standard thermistor (second self-heating type thermistor). Reference numeral 4 is a power supply voltage and reference numeral 5 is a switch for intermittent energization. Reference numeral R1 is a current limit resistance for the detection thermistor 2. Reference numerals R2 and R3 are current limit resistances for the standard thermistor 3. Reference numeral 9 is a differential amplifier circuit for amplifying the output voltage of the detection thermistor 2 and the output voltage of the standard thermistor. Reference numeral 10 is a waveform shaping circuit for converting the output of the differential amplifier circuit 9 into "1" or "0". Reference numeral 11 is a microcomputer (1-chip microcomputer). Reference numeral 12 is a display portion, reference numeral 13 is a buzzer, reference numeral 14 is a communication portion, reference numeral 15 is a memory, and reference numeral 16 is a feed-water valve to supply water (humidifying water) to the humidifying dish portion 30. Reference numeral 17 is a heater (humidifying heater) for heating the inner bottom surface (humidifying dish portion 30) of the incubator. Reference numeral 18 is a heater provided in an area other than the inner bottom surface of the incubator for heating the inside.

In the incubator, for example, 24 volts as the power supply voltage 4 passes through the switch 5 and the current limit resistances R1, R2 and R3 to be applied to the detection thermistor 2 and the standard thermistor 3 of the water level sensor 1.

A resistance of an NTC thermistor is reduced as an ambient temperature thereof increases. Output voltages Vt and Va from the detection thermistor 2 and the standard thermistor 3 have characteristics depending on the ambient temperature, and the presence/absence of water, as shown in Fig. 2. A multipurpose current flows to the detection thermistor 2 to purposely self-heat the detection thermistor. Thus the amount of self-heating of the standard thermistor 3 is less than the amount of self-heating of the detection thermistor 2. When the detection thermistor 2 is present in the water, its discharge characteristic is excellent as compared with when the detection thermistor is present in the air (drought state). Accordingly, the temperature becomes low, the resistance value becomes high and the output voltage Vt becomes high. The standard thermistor 3 has a general characteristic suppressing self-heating and has little variation in accordance with the output voltages in the air and water.

The two output voltages are input to the waveform shaping circuit 10 via the differential amplifier 9. When the output voltage Vt is lower than the output voltage Va, that is, when the water level sensor 1 is present in the air, the waveform shaping circuit 10 outputs output "0" to a port A of the microcomputer 11. When the output voltage Vt is higher than the output voltage Va, that is, when the water level sensor 1 is present in the water, the waveform shaper 10 outputs output "1" to the port A of the microcomputer 11.

Based on the input of the port A, the microcomputer 11 determines that there is no water in the humidifying dish portion 30 in case of the output "0", and that there is the water in the humidifying dish portion 30 in case of the output "1".

The output voltage Va is directly input from a port B of the microcomputer 11 to measure a value thereof by an A/D converter 6 and thus the ambient temperature (water temperature, when there is the water) of the standard thermistor 3 is determined. In addition, data from a storeroom temperature sensor (not shown) for measuring a temperature of a storeroom of the incubator is also input to the microcomputer 11. The microcomputer 11 calculates the humidity (relative humidity) of the storeroom based on the water temperature data and storeroom temperature data and controls energization rates of the humidifying heater 17 and the heater 18 so as to obtain a preliminarily set temperature and humidity.

In this first embodiment, the water level sensor 1 is configured so as to detect the water by using the standard thermistor 3 and the detection thermistor 2. However, only the detection thermistor 2 may be used. In this case, it is preferable that the output voltage Vt of the detection thermistor 2 is read by the A/D converter of the microcomputer and a memory in which the value of the output voltage Vt obtained by the temperature in the storeroom and the presence/absence of the water is written is provided to perform the determination operation based on the value of the memory, the value of the output voltage Vt and the value of the temperature in the storeroom.

Returning to the first embodiment, the incubator will be described. Fig. 3 is a diagram for illustrating the thermistor 2. In Fig. 3, reference numeral 2a is a lead wire. Reference numeral 2b is a temperature sensor. Reference numeral 2c is a protective tube for protecting the periphery of a thermistor element 2d in the temperature sensor 2b. The thermistor element 2d is soldered 2f to the lead wire 2a. A buffer 2i is disposed at the periphery of the thermistor element 2d and a filler 2h is disposed around the lead wire 2a. The thermistor 3 has the same configuration as above.

The protective tube 2c is made of metal which is resistant to ultraviolet radiation, such as a stainless material (SUS316). The protective tube 2c is provided to protect the thermistor from ultraviolet rays. That is, as described in the above Japanese Patent No. 3670876 , there is also an incubator of a type in which a storeroom is irradiated by ultraviolet rays. Accordingly, the protective tube 2c prevents the deterioration of the performance of the thermistor, which occurs by the ultraviolet rays.

Fig. 4 is a perspective view of an example of the attachment of the water level sensor 1. As illustrated in Fig. 4, holes are defined at the height corresponding to a water level position to be detected and the thermistors 2 and 3 of the water level sensor 1 of a humidifying water dish portion 30 disposed in the bottom portion in the storeroom of the incubator are inserted to the holes from the outside. Then, O-rings (not shown) or the like are used to waterproof gaps and fix the thermistors. In this manner, in the humidifying dish portion 30 in the storeroom, only the temperature sensor 2b of the thermistor protrudes so as to suppress the bad effects occurring by ultraviolet rays and the deterioration of maintenance such as cleaning.

Next, automatic water feeding of the first embodiment will be described with reference to Fig. 5.

In an automatic water feeding function of the microcomputer 11, the water is intermittently detected. That is, since the detection thermistor 2 of the water level sensor 1 purposely increases a self-heating value thereof, a lifetime thereof may be affected when the thermistor is continuously energized under a high-temperature environment. Accordingly, the thermistor is intermittently driven at time intervals so as not to interfere with the detection of a water level. That is, the microcomputer 11 of Fig. 1 intermittently turns on the switch 5 to apply a power supply voltage of 24 V to the detection thermistor 2 via the resistance R1. In this manner, energization is performed. When the output voltage Vt of the detection thermistor 2 is balanced by the energization, the water detection is performed. In this first embodiment, the ON state is maintained for 2 minutes and the water detection is performed. After that, the ON state is switched to the OFF state and the OFF state is maintained for 8 minutes and then switched to the ON state again. This operation is repeated.

As described above, the microcomputer 11 turns on the switch 5 and counts 2 minutes. When the 2 minutes are counted, at this time, the presence/absence of the water and the water temperature are detected by the output of the water level sensor 1 and the temperature in the storeroom is detected from the storeroom temperature sensor.

When there is the water, the switch 5 is turned off for 8 minutes, and as described above, the humidifying heater 17 and the heater 18 are controlled so that the humidity and the temperature in the storeroom is adjusted to a predetermined humidity and temperature.

At this time, when there is no water, an automatic water feeding mode is applied. That is, the microcomputer 11 opens the feed-water valve 16 for a predetermined period of time to supply a predetermined amount of water to the humidifying dish portion 30. Herein, when the humidifying dish portion 30 is continuously replenished with the water at one time, the temperature and the humidity in the incubator may vary largely.

Accordingly, in the first embodiment, the humidifying dish portion 30 is replenished with the water by intermittently opening the feed-water valve 16. For example, in this first embodiment 1, when the feed-water valve 16 is opened for 2 minutes and a predetermined amount of water is thereby replenished, the feed-water valve 16 is not continuously opened for 2 minutes. A cycle in which the valve is opened for 1 second and then closed for 4 seconds is repeated 120 times when the feed-water valve 16 supplies the water to the humidifying dish portion 30 during the culture medium is incubated, the feed-water valve 16 decreases the amount of water supplied per unit time than the amount of water supplied during the culture medium is not incubated. That is, when the incubation is not performed, it doesn't matter if a temperature and a humidity vary and thus the water is supplied to shorten the amount of time. However, during the incubation, the water is slowly supplied to suppress the variation in temperature and humidity.

Further, during the intermittent supply of the water, the heater (other than humidifying heater) in the storeroom of the incubator is controlled as normal. The humidifying heater is controlled to be stopped to prevent no-water heating. Then, during the intermittent supply, the humidifying heater in the storeroom is controlled again.

Moreover, during the supply of the water, the drought in the humidifying dish portion 30 (accurately, there is no water at the height of water level sensor) is displayed by the display portion 12, notified by the buzzer, or notified to the outside (mobile phone, outside organization, other room) by the communication portion with the use of an E-mail. In addition, even after the supply of the water is ended, the display portion displays the history (number of times, time) of the drought state.

In this first embodiment, the example of performing the automatic water feeding when the drought is detected is shown. However, the present application can be employed for other incubators without the automatic water feeding function. In this case, when the drought is detected, the microcomputer 11 notifies the drought and stops (or weakens) the heating operation performed by the humidifying heater to prevent no-water heating. In addition, the microcomputer 11 causes the display portion to display the history (number of times, time) of the drought state even after the manual supply of the water is ended by a user. This drought history data is important as compared with the case of the incubator with the automatic water feeding function.

## Claims

1. An incubator for incubating a culture medium, the incubator comprising a water reservoir (30), detection means (1) for detecting when the amount of water present in the water reservoir (30) has dropped below a predetermined level and means for supplying water (16) to said reservoir (30) in response to a signal from said detection means (1) to indicate that the water is below said predetermined level, **characterised in that** said means for supplying water (16) is configured to supply water at a reduced rate whilst a culture medium is being incubated.

2. An incubator according to claim 1, further comprising a heater (17) configured to control the temperature of water in the water reservoir (30).

3. An incubator according to claim 2, wherein the heater (17) is configured to stop or reduce the heat provided when the means for supplying water supplies water to the water reservoir (30).

4. An incubator according to any preceding claim, wherein the water reservoir (30) is disposed in the lower portion of the incubator.

5. An incubator according to any preceding claim, wherein said means for supplying water (16) is configured to supply it intermittently until said predetermined level has been reached, whilst a culture medium is being incubated.

6. An incubator according to claim 5, including control means (11) operable to control the supply of water to said water reservoir.

7. An incubator according to any preceding claim, further comprising a notification means (12,13) configured to notify a user when the amount of water present in the water reservoir (30) has dropped below a predetermined level.

8. An incubator according to claim 7, wherein the notification means (12,13) is configured to store and notify a history of when the amount of water present in the water reservoir (30) has dropped below a predetermined level..

9. An incubator according to claim 1, further comprising an ultraviolet ray radiation means to radiate ultraviolet rays for sterilizing the water; and a temperature sensor to measure a temperature of the water, which is protected from the ultraviolet rays by a tube (2c).

10. An incubator according to claim 9, wherein the temperature sensor comprises a first self-heating type thermistor (2) and a second self-heating type thermistor (3), the level of self heating of the second thermistor (3) being less than the level of self heating of the first thermistor (2), and the thermistors (2,3) being provided at a position corresponding to a predetermined water level of the water reservoir (30), wherein the incubator further comprises a power source configured to supply power to the thermistors intermittently, and a control means (10,11) configured to compare the output voltages corresponding to the temperatures measured by the thermistors (2,3) and to detect that the water level of the water reservoir (30) is below the predetermined water level when the voltage of the first thermistor (2) is less than the voltage of the second thermistor (3).

11. A method of incubating a culture medium in an incubator comprising a water reservoir (30), the method including the steps of detecting when the amount of water present in the water reservoir (30) has dropped below a predetermined level and supplying water to said reservoir (30) in response to a signal from a detection means (1) indicating that the water level has fallen below said predetermined level, **characterised by** the method including the step of supplying water to the water reservoir (30) at a reduced rate when a culture medium is being incubated in the incubator.

12. A method according to claim 11, wherein the method includes the step of supplying water to the water reservoir (30) intermittently over a period of time, when a culture medium is being incubated in the incubator.

## Patentansprüche

1. Inkubator zum Inkubieren eines Kulturmediums, wobei der Inkubator ein Wasserreservoir (30) umfasst, eine Erfassungseinrichtung (1) zum Erfassen, wann die in dem Wasserreservoir (30) vorhandene Wassermenge unter einen vorbestimmten Pegel fällt, sowie eine Einrichtung zur Versorgung des Reservoirs (30) mit Wasser (16) in Antwort auf ein Signal von der Erfassungseinrichtung (1) zur Anzeige, dass das Wasser unter dem vorbestimmten Pegel ist, **dadurch gekennzeichnet, dass** die Einrichtung zur Versorgung mit Wasser (16) dazu ausgelegt ist, Wasser mit einer verringerten Rate bereitzustellen, während ein Kulturmedium inkubiert wird.

2. Inkubator nach Anspruch 1, ferner umfassend einen Heizer (17), der dazu ausgelegt ist, die Temperatur von Wasser in dem Wasserreservoir (30) zu steuern.

3. Inkubator nach Anspruch 2, wobei der Heizer (17) dazu ausgelegt ist, die gelieferte Hitze zu stoppen oder zu verringern, wenn die Einrichtung zur Versorgung mit Wasser das Wasserreservoir (30) mit Wasser versorgt.

4. Inkubator nach einem der vorhergehenden Ansprüche, wobei das Wasserreservoir (30) im unteren Bereich des Inkubators angeordnet ist.

5. Inkubator nach einem der vorhergehenden Ansprüche, wobei die Einrichtung zur Versorgung mit Wasser (16) dazu ausgelegt ist, es intermittierend bereitzustellen, bis der vorbestimmte Pegel erreicht ist, während ein Kulturmedium inkubiert wird.

6. Inkubator nach Anspruch 5, umfassend eine Steuereinrichtung (11), die dazu ausgelegt ist, die Versorgung des Wasserreservoirs mit Wasser zu steuern.

7. Inkubator nach einem der vorhergehenden Ansprüche, ferner umfassend eine Benachrichtigungseinrichtung (12, 13), die dazu ausgelegt ist, einen Benutzer zu benachrichtigen, wenn die in dem Wasserreservoir (30) vorhandene Menge von Wasser unter einen vorbestimmten Pegel gefallen ist.

8. Inkubator nach Anspruch 7, wobei die Benachrichtigungseinrichtung (12, 13) dazu ausgelegt ist, eine Historie dahingehend zu speichern und mitzuteilen, wann die in dem Wasserreservoir (30) vorhandene Menge von Wasser unter einen vorbestimmten Pegel gefallen ist.

9. Inkubator nach Anspruch 1, ferner umfassend eine Ultraviolettstrahlen-Bestrahlungseinrichtung zum Einstrahlen von Ultraviolettstrahlen zum Sterilisieren des Wassers, sowie einen Temperatursensor zum Messen einer Temperatur des Wassers, der durch ein Rohr (2c) vor den Ultraviolettstrahlen geschützt ist.

10. Inkubator nach Anspruch 9, wobei der Temperatursensor einen ersten Thermistor (2) vom selbstheizenden Typ und einen zweiten Thermistor (3) vom selbstheizenden Typ umfasst, wobei der Grad der Selbstheizung des zweiten Thermistors (3) kleiner ist als der Grad der Selbstheizung des ersten Thermistors (2), und wobei die Thermistoren (2, 3) an einer Position entsprechend einem vorbestimmten Wasserpegel des Wasserreservoirs (30) vorgesehen sind, wobei der Inkubator ferner eine Energiequelle umfasst, die dazu ausgelegt ist, den Thermistoren intermittierend Energie bereitzustellen, sowie eine Steuereinrichtung (10, 11), die dazu ausgelegt ist, die Ausgangsspannungen entsprechend den von den Thermistoren (2, 3) gemessenen Temperaturen zu vergleichen und zu erfassen, dass der Wasserpegel des Wasserreservoirs (30) unter dem vorbestimmten Wasserpegel ist, wenn die Spannung des ersten Thermistors (2) kleiner ist als die Spannung des zweiten Thermistors (3).

11. Verfahren zum Inkubieren eines Kulturmediums in einem Inkubator, umfassend ein Wasserreservoir (30), wobei das Verfahren die Schritte umfasst, zu erfassen, wann die in dem Wasserreservoir (30) vorhandene Menge von Wasser unter einen vorbestimmten Pegel fällt, und das Reservoir (30) in Antwort auf ein Signal von einer Erfassungseinrichtung (1) mit Wasser zu versorgen, welches anzeigt, dass der Wasserpegel unter den vorbestimmten Pegel gefallen ist, **dadurch gekennzeichnet, dass** das Verfahren den Schritt umfasst, das Wasserreservoir (30) mit einer verringerten Rate mit Wasser zu versorgen, wenn ein Kulturmedium in dem Inkubator inkubiert wird.

12. Verfahren nach Anspruch 11, wobei das Verfahren den Schritt umfasst, das Wasserreservoir (30) intermittierend über eine Zeitdauer mit Wasser zu versorgen, wenn ein Kulturmedium in dem Inkubator inkubiert wird.

## Revendications

1. Incubateur destiné à mettre en incubation un milieu de culture, l'incubateur comprenant un réservoir d'eau (30), un moyen de détection (1) pour détecter le moment où la quantité d'eau présente dans le réservoir d'eau (30) a baissé au-dessous d'un niveau prédéterminé et un moyen d'alimentation en eau (16) dudit réservoir (30) en réponse à un signal provenant dudit moyen de détection (1) pour indiquer que l'eau est au-dessous dudit niveau prédéterminé, **caractérisé en ce que** ledit moyen d'alimentation en eau (16) est configuré pour l'alimentation en eau à un débit réduit, tandis qu'un milieu de culture est mis en incubation.

2. Incubateur selon la revendication 1, comprenant en outre un dispositif de chauffage (17) configuré pour réguler la température de l'eau dans le réservoir d'eau (30).

3. Incubateur selon la revendication 2, dans lequel le dispositif de chauffage (17) est configuré pour arrêter ou réduire la chaleur fournie lorsque le moyen d'alimentation en eau alimente en eau le réservoir d'eau (30).

4. Incubateur selon l'une des revendications précédentes, dans lequel le réservoir d'eau (30) est disposé dans la partie inférieure de l'incubateur.

5. Incubateur selon l'une des revendications précédentes, dans lequel ledit moyen d'alimentation en eau (16) est configuré pour l'alimenter par intermittence jusqu'à ce que ledit niveau prédéterminé soit atteint, tandis qu'un milieu de culture est mis en incubation.

6. Incubateur selon la revendication 5, comprenant un moyen de commande (11) opérable pour commander l'alimentation en eau dudit réservoir d'eau.

7. Incubateur selon l'une des revendications précédentes, comprenant en outre un moyen de notification (12, 13) configuré pour avertir un utilisateur lorsque la quantité d'eau présente dans le réservoir d'eau (30) a baissé au-dessous d'un niveau prédéterminé.

8. Incubateur selon la revendication 7, dans lequel le moyen de notification (12, 13) est configuré pour stocker et notifier un historique des moments où la quantité d'eau présente dans le réservoir d'eau (30) a baissé au-dessous d'un niveau prédéterminé.

9. Incubateur selon la revendication 1, comprenant en outre un moyen de rayonnement ultraviolet pour faire irradier des rayons ultraviolets stérilisant l'eau ; et un capteur de température pour mesurer la température de l'eau, qu'un tube (2c) protège des rayons ultraviolets.

10. Incubateur selon la revendication 9, dans lequel le capteur de température comprend une première thermistance (2) de type à auto-chauffage et une deuxième thermistance (3) de type à auto-chauffage, le niveau d'auto-chauffage de la deuxième thermistance (3) étant inférieur au niveau d'auto-chauffage de la première thermistance (2), et les thermistances (2, 3) étant prévues à une position correspondant à un niveau d'eau prédéterminé du réservoir d'eau (30), l'incubateur comprenant en outre une source d'alimentation configurée pour alimenter en puissance les thermistances par intermittence, et un moyen de commande (10, 11) configuré pour comparer les tensions de sortie correspondant aux températures mesurées par les thermistances (2, 3) et pour détecter que le niveau d'eau du réservoir d'eau (30) est inférieur au niveau d'eau prédéterminé lorsque la tension de la première thermistance (2) est inférieure à la tension de la deuxième thermistance (3).

11. Procédé d'incubation d'un milieu de culture dans un incubateur comprenant un réservoir d'eau (30), le procédé comprenant les étapes consistant à détecter les moments où la quantité d'eau présente dans le réservoir d'eau (30) a baissé au-dessous d'un niveau prédéterminé et à alimenter en eau ledit réservoir (30) en réponse à un signal provenant d'un moyen de détection (1) indiquant que le niveau d'eau a baissé au-dessous dudit niveau prédéterminé, le procédé étant **caractérisé par** le fait de comprendre l'étape consistant à alimenter en eau le réservoir d'eau (30) à un débit réduit lorsqu'un milieu de culture est mis en incubation dans l'incubateur.

12. Procédé selon la revendication 11, le procédé comprenant l'étape consistant à alimenter en eau le réservoir d'eau (30) par intermittence pendant une période, lorsqu'un milieu de culture est mis en incubation dans l'incubateur.
